# EUROPÄISCHE PATENTSCHRIFT

(11) **EP 1 358 306 B1**
(45) Veröffentlichungstag und Bekanntmachung des Hinweises auf die Patenterteilung: **22.09.2004**
(21) Anmeldenummer: 02782450.7
(22) Anmeldetag: 02.07.2002
(51) Int. Cl.: C11C 3/04, C11C 3/10

(54) **VERFAHREN ZUR UMESTERUNG VON FETT UND/ODER ÖL MITTELS ALKOHOLYSE**
METHOD FOR TRANSESTERIFICATION OF FATS AND/OR OILS BY MEANS OF ALCOHOLYSIS
PROCEDE DE TRANSESTERIFICATION DE MATIERE GRASSE ET/OU D'HUILE PAR ALCOOLYSE

(30) Priorität: 06.07.2001 DE 10132842
(43) Veröffentlichungstag der Anmeldung: 05.11.2003
(73) Patentinhaber: Peter, Siegfried, Prof. Dr., 91080 Uttenreuth-Weiher (DE)
(72) Erfinder: PETER, Siegfried, 91080 Uttenreuth (DE); WEIDNER, Eckhard, 44795 Bochum (DE)
(74) Vertreter: Beyer, Andreas, Dr.
(86) Internationale Anmeldenummer: PCT/EP2002/007311
(87) Internationale Veröffentlichungsnummer: WO 2003/004591

(56) Entgegenhaltungen:
- WO-A-01/12581
- CA-A- 2 131 654
- GB-A- 575 416
- SERCHELI ET AL: "Alkylguanidine-catalyzed heterogeneous transesterification of soybean oil" JOURNAL OF THE AMERICAN OIL CHEMISTS' SOCIETY, AMERICAN OIL CHEMISTS' SOCIETY. CHAMPAIGN, US, Bd. 76, Nr. 10, 1999, Seiten 1207-1210, XP002163432 ISSN: 0003-021X

## Beschreibung

Die Erfindung betrifft ein Verfahren zur Gewinnung von Fettsäureestern aus Triacylglyceriden mittels Alkoholyse. Insbesondere betrifft die Erfindung ein Verfahren zur Umesterung von Fett und/oder Öl durch Alkoholyse, bei welchem zur Beschleunigung des Verfahrens in der Anfangsphase mindestens ein Alkanolfettsäureester in einer solchen Menge zugegeben wird, dass das dadurch entstehende Reaktionsgemisch einphasig wird.

Umesterungsreaktionen an sich sind bekannt. Sie stellen eine kommerziell bedeutsame Klasse industrieller organischer Reaktionen dar. Bei einer Umesterungsreaktion wird ein Ester durch Austausch der Säuregruppen oder durch Austausch der alkoholischen Gruppen in einen anderen Ester überführt. Wird die Umesterung durch Austausch der alkoholischen Gruppen vorgenommen, spricht man von der sogenannten Alkoholyse (auch Alkanolyse). Bei der Alkoholyse wird der Alkohol bzw. das Alkanol im Überschuss zugesetzt, um eine hohe Ausbeute am gewünschten Ester zu erhalten. In neuerer Zeit hat im Zusammenhang mit der Erzeugung von Dieselkraftstoff aus nachwachsenden Rohstoffen die Herstellung von Alkyfestern, insbesondere von Methylestern, durch vegetabilische Öle (z.B. Rapsöl, Sojaöl), erheblich an Aktualität gewonnen.

Die Umesterung ist eine Gleichgewichtsreaktion, die in der Regel bereits durch Mischen der Reaktanden ausgelöst wird. Die Reaktion verläuft jedoch so langsam, dass für die kommerzielle Durchführung der Reaktion in der Regel ein Katalysator erforderlich ist. Als Katalysatoren dienen üblicherweise starke Säuren oder starke Basen.

Fette und Öle bestehen überwiegend aus Glyceriden (Mono-, Di- und Triglyceriden). Bei der Umesterung solcher Fette und Öle kann die Komponente Glycerin durch niedermolekulare einwertige Alkohole substituiert werden. In der Praxis wird hierzu häufig die Methode nach Bradshaw (beschrieben in den US-Patenten 2,271,619 und 2,360,844) angewandt. Die Reaktion wird in einem offenen Behälter, der aus gewöhnlichem Kohlenstoffstahl bestehen kann, durchgeführt. Das Fett oder Öl muss trocken (wasserfrei), sauber und vor allem neutral sein, d.h. der Gehalt an freien Fettsäuren muss vernachfässigbar gering sein (Säurezahl nicht höher als 1,5). Im allgemeinen wird zur Erhöhung der Ausbeute und der Reaktionsgeschwindigkeit dem Reaktionsgemisch der einwertige Alkohol in hohem Überschuss zugesetzt (das Äquivalentverhältnis ist oft höher als 1:6).

In einer Arbeit von Wright et al. (H.J. Wright, J.B. Segur, H.V. Clark, S.K. Coburn, E.E. Langdon and R.N. DuPuis, Oil & Soap, 21 [1944] 145 - 148) wurden die genauen Bedingungen für die Alkoholyse von Fetten mit Methanol und Ethanol im Detail untersucht. Weiterhin wird von den Autoren über Experimente über die Alkoholyse mit anderen einwertigen Alkoholen berichtet. Es wird dargelegt, dass die oben beschriebene, mit Alkali katalysierte Alkoholyse nur dann völlig erfolgreich ist, wenn das Fett nahezu frei von freien Fettsäuren und das Reaktionsgemisch frei von Wasser ist. Ist eine dieser Bedingungen nicht erfüllt, kommt es zur Verseifung, die einen Verlust an Alkalinität und die Bildung einer Gelstruktur zur Folge hat, die die Abtrennung und das Absetzen des Glycerins verhindert oder verlangsamt.

Die Umesterung der Triacylglyceride mittels Alkoholyse ist dadurch gekennzeichnet, dass die Reaktion zwischen Alkanol und Triacylglyceriden einer Anlaufphase bedarf, in der nur eine geringe Reaktionsgeschwindigkeit besteht, weil die Reaktionskomponente Alkanol nicht im Öl löslich ist. Besonders bei der Herstellung von Methylestern ist dieser Umstand sehr störend, da Methanol nur wenig löslich in den umzuesternden Ölen und Fetten ist. Dagegen ist Methanol in den Methylestern der Fettsäuren gut löslich. Wegen der geringen Methanolkonzentration im Öl läuft die Umesterungsreaktion nur langsam ab. Die Reaktionsmischung muss kräftig durchmischt werden bis schließlich der Gehalt an Estern soweit angestiegen ist, dass das Reaktionsgemisch einphasig wird und die Reaktionsgeschwindigkeit plötzlich stark ansteigt.

Als Katalysatoren werden in der Praxis Alkalimetalle oder Alkoholate der Alkalimetalle verwendet. Die alkalischen Katalysatoren lösen sich im Reaktionsgemisch auf, d.h. die Reaktion wird homogen katalysiert. Die Alkalimetalle und ihre Alkoholate werden im Verlaufe der Reaktion zu Seifen umgesetzt, die sich besonders im entstehenden Glycerin lösen und dessen Weiterverarbeitung zu reinem Glycerin verteuern. Aber auch im Methylester verbleiben geringe Mengen an Alkali, die bei der Verwendung der Methylester als Dieselkraftstoff nicht ganz problemlos sein dürften. Deswegen wurden in neuerer Zeit auch heterogen katalysierte Verfahren, z.B. unter Verwendung eines Metallsalzes einer stark basischen Aminosäure als festen im Reaktionsgemisch unlöslichen Katalysator vorgeschlagen (Patentanmeldung DE 199 50 593. A1).

Ferner wurde ein Katalysator auf Basis von Titanoxiden entwickelt, der den Nachteil hat, dass die Reaktionstemperaturen im Bereich von 240°C liegen.

Ausgehend von diesem Stand der Technik ist es die Aufgabe der Erfindung diese Anlaufphase bei mäßigen Reaktionstemperaturen zu eliminieren bzw. zu verkürzen und das Verfahren damit effektiver zu gestalten.

Die Lösung dieser Aufgabe erfolgt durch ein Verfahren zur Umesterung von Fett und/oder Öl mittels Alkoholyse, bei welchem dem umzuesternden Fett und/oder Öl zur Durchführung der Alkoholyse ein Alkanol, insbesondere ein einwertiges Alkanol im Überschuss zugegeben wird, dadurch gekennzeichnet, dass dem Fett und/oder Öl mindestens ein Alkanolfettsäureester in einer solchen Menge zugegeben wird, dass das dadurch entstehende Reaktionsgemisch bei Reaktionsbedingungen einphasig wird.

Überraschenderweise wurde nun gefunden, dass schon eine recht kleine Menge an zugeführten Alkanolfettsäureestern diese Aufgabe lösen kann. Die Zugabe der Alkanolfettsäureester kann dabei vor, nach oder gleichzeitig mit der Zugabe des Alkanols stattfinden.

Nach dem Verfahren dieser Erfindung wird die Anfangsphase bei der Umesterung dadurch vermieden bzw. verkürzt, dass beispielsweise für den Fall der Alkoholyse mit Methanol ein Teil der kontinuierlich erzeugten Methylester dem Ausgangsprodukt an Triacylglyceriden in solchen Mengen zugesetzt wird, dass das Gemisch aus Öl, Methanol und Methylester einphasig wird.
Wenn das Reaktionsgemisch in einer einzigen Phase vorliegt, ist die wirksame Alkanolkonzentration von Anfang an hoch und die Reaktion läuft entsprechend schnell ab. Beispielsweise wurde bei 135°C in der Anfangsphase eines mit Zinkarginat heterogen katalysierten Prozesses (Methylesterherstellung aus Palmöl) eine Reaktionsgeschwindigkeit von 0,8 g/skg_{Znarg} und nach Eintritt von Einphasigkeit eine Reaktionsgeschwindigkeit von 2,5 g/skg_{Znarg} gemessen.

Das Fett und/oder Öl, welches in dem erfindungsgemäßen Verfahren verwendet wird, kann insbesondere biologischen Ursprungs sein.

Die Menge an Alkanolfettsäureestern, die zur Herstellung eines einphasigen Gemisches zugegeben werden muss, hängt von der Qualität des Öles, der Höhe des Alkanolüberschusses und der Reaktionstemperatur ab. Der Alkanolüberschuss wird zur Erhöhung der Reaktionsgeschwindigkeit und der Ausbeute an Fettsäurealkanolestern im allgemeinen mit einem Äquivalentverhältnis (d.h. Verhältnis von Mol Fettsäuren im Fett und/oder Öl zu Mol einwertigem Alkanol) von 1:6 oder darüber angewendet.

Alkanolfettsäureester, welche in dem Verfahren vorzugsweise zugegeben werden, sind z.B. Methylester, Ethylester und/oder Propylester.

Die Alkanolfettsäureester werden vorzugsweise in einer Menge von 5 bis 50 Gew.-%, besonders bevorzugt 12 bis 20 Gew.-% bezogen auf das Fett und/oder Öl zugegeben.

Das erfindungsgemäße Verfahren ist besonders wirkungsvoll, wenn beabsichtigt ist, die Umesterung in einem heterogen katalysierten Prozess, welcher vorzugsweise kontinuierlich sein kann, zu betreiben. Aber auch bei einem homogen katalysierten Verfahren ist das Verfahren nach dieser Erfindung vorteilhaft, weil die Kosten für die Verwirbelung der beiden Phasen in der Anfangsphase der Reaktion gespart werden können. Solche heterogen katalysierten Verfahren werden beispielsweise in der oben erwähnten DE 199 50 593 beschrieben.

So wird in einer weiteren bevorzugten Ausführungsform dem Verfahren ein Katalysator zugesetzt, welcher entweder ein löslicher Katalysator oder ein in Alkanolen und im Reaktionsgemisch unlösliches Metallsalz einer Aminosäure oder eines Aminosäurederivats sein kann.

Der gelöste Katalysator kann beispielsweise in gelösten Alkalimetallen oder Alkoholaten von Alkalimetallen bestehen.

Der unlösliche Katalysator kann eine Metallkomponente aufweisen, die Calcium, Strontium, Barium, ein anderes Erdalkalimetall, oder ein Schwermetall, insbesondere Silber, Kupfer, Zink, Mangan, Eisen, Nickel, Kobalt, Lanthan oder ein anderes Seltenerdmetall ist, während die Aminosäurekomponente des unlöslichen Katalysators quarternären Stickstoff oder eine Guanidinogruppe enthalten kann. Besonders bevorzugt ist der unlösliche Katalysator ein Schwermetallsalz des Arginins, insbesondere das Zinksalz oder das Cadmiumsalz des Arginins. Die im Reaktionsgemisch unlöslichen, katalytisch aktiven Salze können dabei auf einem geeigneten Träger niedergeschlagen sein.

Das erfindungsgemäße Verfahren lässt sich besonders effektiv durchführen, wenn der Anteil an freien Fettsäuren in dem umzuesterndem Fett und/oder Öl weniger als 0,5 Gew.-%, insbesondere weniger als 0,1 Gew.-% beträgt.

Es wurde außerdem gefunden, dass die Reaktionstemperaturen bei der heterogen katalysierten Umesterung vorzugsweise im Bereich von 80 bis 160°C, insbesondere im Bereich von 100 bis 150°C liegen sollten.

Besonders bevorzugt wird in dem erfindungsgemäßen Verfahren ein Vorgehen, bei dem die Alkanolfettsäureester rezirkuliert werden, die nach Abtrennung des Glycerins aus dem Produktstrom bei der anschließenden Abtrennung und Reinigung der Hauptmenge der erzeugten Methylester durch Destillation als Sumpfprodukt zurückbleiben. Auf diese Weise werden gleichzeitig geringe Mengen an nicht umgesetzten Glyceriden rezirkuliert. Außerdem wird dadurch der Glyceringehalt in der Endphase der Reaktion herabgesetzt und die Ausbeute der Gleichgewichtsreaktion entsprechend erhöht. Insgesamt wird so eine kontinuierliche Prozessführung ermöglicht.

Die Menge an Methylestern, die zur Herstellung von Einphasigkeit bei Reaktionstemperaturen im Bereich von 100 bis 150°C bevorzugt ist, beträgt ca. 12 bis 20 Gew.-%.

Nachfolgend wird das erfindungsgemäße Verfahren an verschiedenen Beispielen näher erläutert.

So wurde das erfindungsgemäße Verfahren an einer Mischung aus Sonnenblumenöl und Methanol getestet. In diesem Fall genügte bei 135°C und einem Äquivalentverhältnis von Mol Fettsäuren im Öl zu Methanol von 1:6 (60 Gew.-% Sonnenblumenöl und 40 Gew.-% Methanol) ein Zusatz von ca. 15% Gew.-% Methylester bezogen auf das Öl, um ein Einphasensystem zu erzeugen. Der sich einstellende Druck betrug im beschriebenen Fall 5 bar. Als Katalysator diente Zinkarginat. Die Reaktionsgeschwindigkeit betrug 2,5 g/skg_{Znarg}. In diesem Beispiel wurde von vornherein eine hohe Reaktionsgeschwindigkeit erhalten.

Außerdem wurde Palmöl bei 150°C mit Methanol im Äquivalentverhältnis von 1:6 vermischt und Zinkarginat als Katalysator zugegeben. Nach Zugabe von 20 Gew.-% Methylester bezüglich Palmöl war das Gemisch einphasig. Die Reaktionsgeschwindigkeit war mit 3,2 g/skg_{Znarg} von vornherein hoch. Die Anfangsphase mit niedriger Reaktionsrate wurde übersprungen.

Palmöl wurde weiterhin bei 85°C mit Methanol im Äquivalentverhältnis von 1:6 vermischt und Zinkarginat als Katalysator zugegeben. Die Reaktionsgeschwindigkeit betrug 0,05 g/skg_{Znarg.} Nachdem durch Zugabe von Methylester (ca. 13 Gew.-% bezüglich Öl) das Reaktionsgemisch einphasig war, wurde bei Umgebungsdruck eine Reaktionsgeschwindigkeit von 0,35 g/skg_{Znarg} gemessen.

Bei Reaktionstemperaturen von 200 bis 240°C werden nach dem in der deutschen Patentschrift DE 198 03 053 C1 beschriebenen Verfahren mit Zinkseifen als Katalysatoren bei Drücken bis zu 90 bar Triglyceride bei hohem äquivalenten Überschuss an Methanol (Äquivalentverhältnis höher als 1:6) zu Estern umgesetzt. Unter diesen Bedingungen ist ein höherer Methylestergehalt notwenig, um ein Einphasensystem herzustellen, als in dem genannten Beispiel bei 135°C.

## Patentansprüche

1. Verfahren zur Umesterung von Fett und/oder Öl mittels Alkoholyse, bei welchem dem umzuesternden Fett und/oder Öl zur Durchführung der Alkoholyse ein Alkanol, insbesondere ein einwertiges Alkanol im Überschuss zugegeben wird, **dadurch gekennzeichnet, dass** dem Fett und/oder Öl mindestens ein Alkanolfettsäureester in einer solchen Menge zugegeben wird, dass das dadurch entstehende Reaktionsgemisch einphasig wird.

2. Verfahren nach Anspruch 1, **dadurch gekennzeichnet, dass** der zugegebene Alkanolfettsäureester ausgewählt wird aus der Gruppe, bestehend aus Methylestern, Ethylestern und/oder Propylestern.

3. Verfahren nach Anspruch 1 oder 2, **dadurch gekennzeichnet, dass** der Alkanolfettsäureester in einer Menge von 5 bis 50 Gew.-%, vorzugsweise 12 bis 20 Gew.-% bezogen auf das Fett und/oder Öl zugegeben wird.

4. Verfahren nach einem der Ansprüche 1 bis 3, **dadurch gekennzeichnet, dass** zur Durchführung der Reaktion ein im Reaktionsgemisch löslicher Katalysator zugegeben wird.

5. Verfahren nach einem der Ansprüche 1 bis 3, **dadurch gekennzeichnet, dass** zur Durchführung der Reaktion dem Reaktionsgemisch ein in Alkanolen und im Reaktionsgemisch unlösliches Metallsalz einer Aminosäure oder eines Aminosäurederivates zugesetzt wird.

6. Verfahren nach Anspruch 4, **dadurch gekennzeichnet, dass** die Alkoholyse durch gelöste Alkalimetalle oder Alkoholate der Alkalimetalle katalysiert wird.

7. Verfahren nach Anspruch 5, **dadurch gekennzeichnet, dass** die Metallkomponente des Katalysators Calcium, Strontium, Barium, ein anderes Erdalkalimetall oder ein Schwermetall, insbesondere Silber, Kupfer, Zink, Mangan, Eisen, Nickel, Kobalt, Lanthan oder ein anderes Seltenerdmetall ist.

8. Verfahren nach einem der Ansprüche 5 oder 7, **dadurch gekennzeichnet, dass** die Aminosäurekomponente des Katalysators quarternären Stickstoff oder eine Guanidinogruppe enthält.

9. Verfahren nach einem der Ansprüche 5 oder 7, **dadurch gekennzeichnet, dass** der Katalysator ein Schwermetallsalz des Arginins, insbesondere das Zinksalz oder das Cadmiumsalz des Arginins ist.

10. Verfahren nach einem der Ansprüche 1 bis 9, **dadurch gekennzeichnet, dass** der Anteil freier Fettsäuren in dem umzuesterndem Fett und/oder Öl weniger als 0,5 Gew.-%, insbesondere weniger als 0,1 Gew.-% beträgt.

11. Verfahren nach einem der Ansprüche 1 bis 10, **dadurch gekennzeichnet, dass** die Umesterung bei Temperaturen in dem Bereich von 80 bis 160°C, vorzugsweise in dem Bereich von 100 bis 150°C durchgeführt wird.

12. Verfahren nach einem der Ansprüche 1 bis 11, **dadurch gekennzeichnet, dass** der dem Reaktionsgemisch zugesetzte Alkanolfettsäureester aus dem bei der Umesterung resultierenden Produktstrom in das Verfahren rezirkuliert wird.

## Claims

1. A process for the transesterification of fat and/or oil by means of alcoholysis wherein, in order to carry out the alcoholysis, an alkanol, in particular a monohydric alkanol, is added in excess to the fat and/or oil to be transesterified, **characterized in that** at least one alkanol fatty acid ester is added to the fat and/or oil in a quantity such that the reaction mixture produced thereby consists of one phase.

2. The process according to claim 1, **characterized in that** the alkanol fatty acid ester added is selected from the group consisting of methyl esters, ethyl esters and/or propyl esters.

3. The process according to claim 1 or 2, **characterized in that** the alkanol fatty acid ester is added in a quantity of 5 to 50 wt.%, preferably 12 to 20 wt.%, based on the fat and/or oil.

4. The process according to one of claims 1 to 3, **characterized in that** a catalyst which is soluble in the reaction mixture is added in order to carry out the reaction,.

5. The process according to one of claims 1 to 3, **characterized in that**, in order to carry out the reaction, a metal salt of an amino acid or of an amino acid derivative which is insoluble in alkanols and in the reaction mixture is added to the reaction mixture.

6. The process according to claim 4, **characterized in that** the alcoholysis is catalyzed by dissolved alkali metals or by alcoholates of the alkali metals.

7. The process according to claim 5, **characterized in that** the metal component of the catalyst is calcium, strontium, barium, another alkaline-earth metal or a heavy metal, in particular silver, copper, zinc, manganese, iron, nickel, cobalt, lanthanum or another rare-earth metal.

8. The process according to one of claims 5 or 7, **characterized in that** the amino add component of the catalyst contains quaternary nitrogen or guanidine group.

9. The process according to one of claims 5 or 7, **characterized in that** the catalyst is a heavy metal salt of arginine, in particular the zinc salt or the cadmium salt of arginine.

10. The process according to one of claims 1 to 9, **characterized in that** the content of free fatty acids in the fat and/or oil to be transesterified is less than 0.5 wt.%, in particular less than 0.1 wt.%.

11. The process according to one of claims 1 to 10, **characterized in that** the transesterification is carried out at temperatures within the range of 80°C to 160°C, preferably within the range of 100°C to 150°C.

12. The process according to one of claims 1 to 11, **characterized In that** the alkanol fatty acid ester added to the reaction mixture is recirculated to the process from the product flow resulting from the transesterification.

## Revendications

1. Procédé de transestérification de matière grasse et/ou d'huile par alcoolyse, dans le cadre duquel un alcanol, en particulier un alcanol monovalent, est additionné en excès à la matière grasse et/ou à l'huile à transestérifier pour réaliser l'alcoolyse, **caractérisé en ce qu'**au moins un ester d'acides gras de l'alcanol est additionné à la matière grasse et/ou à l'huile dans une quantité telle que le mélange de réaction ainsi obtenu devient monophasique.

2. Procédé selon la revendication 1, **caractérisé en ce que** l'ester d'acides gras de l'alcanol additionné est sélectionné dans le groupe constitué par les esters méthyliques, les esters éthyliques et/ou les esters propyliques.

3. Procédé selon la revendication 1 ou 2, **caractérisé en ce que** l'ester d'acides gras de l'alcanol est additionné dans une quantité représentant 5 à 50 % en poids, de préférence 12 à 20 % en poids de la matière grasse et/ou de l'huile.

4. Procédé selon l'une des revendications 1 à 3, **caractérisé en ce qu'**un catalyseur soluble dans le mélange de réaction est additionné pour réaliser la réaction.

5. Procédé selon l'une des revendications 1 à 3, **caractérisé en ce qu'**un sel métallique d'un acide aminé ou d'un dérivé d'acides aminés, lequel est insoluble dans le mélange de réaction et dans les alcanols, est additionné au mélange de réaction.

6. Procédé selon la revendication 4, **caractérisé en ce que** l'alcoolyse est catalysée par des métaux alcalins dissous ou des alcoolats dissous et issus des métaux alcalins.

7. Procédé selon la revendication 5, **caractérisé en ce que** la composante métallique du catalyseur consiste en du calcium, strontium, baryum, un autre métal alcalino-terreux ou un métal lourd, en particulier l'argent, le cuivre, le zinc, le manganèse, le fer, le nickel, le cobalt, le lanthane ou un autre métal du groupe des terres rares.

8. Procédé selon l'une des revendications 5 ou 7, **caractérisé en ce que** la composante acide aminé du catalyseur contient de l'azote quarternaire ou un groupement guanidino.

9. Procédé selon l'une des revendications 5 ou 7, **caractérisé en ce que** le catalyseur est un sel de métal lourd de l'arginine, en particulier le sel de zinc ou le sel de cadmium de l'arginine.

10. Procédé selon l'une des revendications 1 à 9, **caractérisé en ce que** la part d'acides gras libres dans la matière grasse et/ou l'huile à transestérifier est inférieure à 0,5 % en poids, en particulier inférieure à 0,1 % en poids.

11. Procédé selon l'une des revendications 1 à 10, **caractérisé en ce que** la transestérification est réalisée à des températures situées entre 80 et 160°C, de préférence entre 100 à 150°C.

12. Procédé selon l'une des revendications 1 à 11, **caractérisé en ce que** l'ester d'acides gras de l'alcanol additionné au mélange de réaction issu du flux de produit résultant de la transestérification et est remis en circulation dans le procédé.
